# EUROPEAN PATENT APPLICATION

(11) **EP 4 497 432 A1**
(43) Date of publication of application: **29.01.2025**
(21) Application number: 23775308.2
(22) Date of filing: 22.03.2023
(51) Int. Cl.: A61K 9/00, A61K 47/28, A61K 47/42, A61K 47/12, A61K 38/00, A61P 3/10, A61P 3/04, A61P 19/10, A23L 33/10, A23L 33/17

(54) **PROTEIN DELIVERY COMPOSITION CONTAINING BILE SALT AND CATIONIC PEPTIDE AND USE THEREOF**

(30) Priority: 22.03.2022 KR 20220035239; 27.01.2023 KR 20230011287
(71) Applicant: Bion Inc., Pohang-si, Gyeongsangbuk-do 37666 (KR)
(72) Inventor: KIM, Jaeyoon, Pohang-si Gyeongsangbuk-do 37855 (KR); JO, Hyungeun, Ulsan 44215 (KR); CHOI, Jungran, Pohang-si Gyeongsangbuk-do 37637 (KR); JEONG, Eunhye, Pohang-si Gyeongsangbuk-do 37693 (KR)
(74) Representative: Herrero & Asociados, S.L.
(86) International application number: PCT/KR2023/003811
(87) International publication number: WO 2023/182812

(57) **Abstract**

The present invention relates to a protein delivery composition containing bile salt and a cationic peptide, and a use thereof. The protein delivery composition has the technical characteristic in that the composition can significantly increase the penetration of the target protein or target peptide to be administered through epithelial tissues, allowing for the effective delivery of the target protein into the body in a non-invasive manner. In addition, the bile salt and cationic peptide can be used in therapeutics for metabolic diseases, health functional foods, and cosmetics.

## Description

### Technical Field

The present disclosure relates to a composition for protein delivery that enhances the absorption rate of target proteins in vivo.

### Background Art

Proteins perform various functions within the body, possess excellent target specificity, exhibit outstanding therapeutic effects, and have fewer side effects, thus finding applications as therapeutic agents for major diseases such as cancer and diabetes. Depending on the characteristics of the pharmaceutical composition, therapeutics are administered via various routes such as injection, oral, inhalation, or mucosal delivery. However, protein therapeutics, being macromolecules, are mainly delivered through invasive methods such as injection, limiting their administration within the body. Therefore, there is a need for methods to administer protein therapeutics in a non-invasive manner for various diseases, including intractable diseases requiring long-term treatment.

Epithelial tissues form the outer covering of all animal body skin, line body cavities and hollow organs, and are the major tissue in glands such as endocrine and exocrine glands. Epithelial tissue protects the body from the invasion of external substances (e.g., pathogens, external proteins, DNA, etc.) or external stimuli, accepts and transmits external stimuli, and is responsible for the transport and exchange of small substances, performing various functions depending on the tissue. However, the delivery efficiency of proteins through epithelial tissue is very low. Therefore, various studies are being conducted on enhancing absorption using bile acids, fatty acids, or sodium salcaprozate (SNAC) to improve the delivery efficiency of protein therapeutics through skin tissue, gastrointestinal tract, respiratory tract, or reproductive mucosa.

Bile salts are substances produced in the gallbladder that aid in the dissolution of fats in the digestive tract, contributing to fat digestion and absorption. They are ionic amphiphilic compounds with a steroid skeleton. In the liver, enzymatic oxidation, isomerization, and reduction of cholesterol form cholate, which is structurally similar to the cholesterol parent molecule. Cholate then chemically binds to one of two amino acids (taurine or glycine) to form "conjugated" cholic acids (L-glycocholate and taurocholate). During digestion, the conjugated cholic acid molecules thus released undergo further chemical modifications (dehydroxylation and deconjugation) by enzymes produced by the intestinal microbiota, generating various bile acids (Molecules 2015, 20, 14451-14473). Bile acids are classified into trihydroxy bile salts (taurocholate, glycocholate, cholate) or dihydroxy bile salts (taurodeoxycholate, glycodeoxycholate, chenodeoxycholate, deoxycholate) depending on their hydroxylation and conjugation status.

Previous studies have utilized bile acids to enhance the delivery efficiency of protein therapeutics in vivo. It has been confirmed that conjugated bile salts such as taurocholate or glycocholate effectively deliver peptides like insulin and salmon calcitonin across the blood-brain barrier, skin, mucosa, cornea, and intestinal mucosa. However, most previous studies involved 1) bile salts acting as one component of liposomes, nanoparticles, or bilosomes rather than their single effect, or 2) experiments conducted in cell models mimicking trans-epithelial delivery or direct delivery to the duodenum, excluding digestion in the stomach. There are almost no results showing efficacy in normal conditions at the organism level, suggesting that bile salts alone do not significantly enhance the delivery of peptides and proteins in vivo.

Thus, there is a rising need for new delivery vehicles with superior in vivo delivery efficiency for target peptides or proteins.

### Disclosure of Invention

### Technical Problem

Leading to the present disclosure, intensive and thorough research conducted by the present inventors with the aim of developing a protein delivery composition capable of enhancing the permeability of target proteins in epithelial tissue resulted in the finding that using a composition containing bile salts and cationic peptides of a specific sequence, optionally with the additional inclusion of specific fatty acids, can enhance the permeability of target proteins in epithelial tissue.

Accordingly, the present disclosure aims to provide a composition for protein delivery with enhanced permeability in epithelial tissue.

Also, the present disclosure is to provide a transdermal absorption agent containing the composition for protein delivery.

Furthermore, the present disclosure is to provide a pharmaceutical composition for the prevention or treatment of metabolic diseases, including the composition for protein delivery.

Moreover, the present disclosure is to provide a health functional food composition including the composition for protein delivery.

Yet moreover, the present disclosure is to provide a cosmetic composition including the composition for protein delivery.

### Solution to Problem

An aspect of the present disclosure provides a composition for protein delivery, the composition including, as an active ingredient, at least one selected from the group consisting of bile salts and peptides represented by SEQ ID NOS: 1 to 34.

The present inventors have diligently researched to develop a composition for protein delivery that can enhance the permeability of target proteins in epithelial tissue. As a result, it was discovered that using a composition containing bile salts and cationic peptides of a specific sequence, optionally with the additional inclusion of specific fatty acids, can enhance the permeability of target proteins in epithelial tissue.

The sequences of SEQ ID NOS: 1 to 34 of the present disclosure are given in Table 1 below.

**TABLE 1**

| SEQ ID NO: | sequence | SEQ ID NO: | sequence | SEQ ID NO: | sequence | SEQ ID NO: | sequence |
|---|---|---|---|---|---|---|---|
| 1 | RRRRR | 10 | RKRKRR | 19 | kk | 28 | KKK |
| 2 | RRRRRR | 11 | KFKWPW | 20 | kkk | 29 | KKKK |
| 3 | RRRRRRR | 12 | KFKWPR | 21 | kkkk | 30 | KKKKK |
| 4 | RRRRRRRRR | 13 | RWRWRW | 22 | kkkkk | 31 | KKKKKKK |
| 5 | rrrrr | 14 | kkkkkk | 23 | kkkkkkk | 32 | KKKKKKKK |
| 6 | rrrrrr | 15 | rkrkrr | 24 | kkkkkkkk | 33 | KKKKKKKKK |
| 7 | rrrrrrr | 16 | kfkwpw | 25 | kkkkkkkkk | 34 | KKKKKKKKKK |
| 8 | rrrrrrrr | 17 | krkwpr | 26 | kkkkkkkkkk | | |
| 9 | KKKKKK | 18 | rwrwrw | 27 | KK | | |

In describing the peptide sequences herein, uppercase letters indicate L-amino acids, and lowercase letters indicate D-amino acids. The amino acid sequences of the present disclosure may be sequences in which L-form amino acids or D-form amino acids are mixed.

The composition for protein delivery of the present disclosure is technically characterized by including a bile salt and at least one peptide selected from the group consisting of peptides represented by SEQ ID NOS: 1 to 34. To enhance the epithelial permeability of a target protein requiring in vivo delivery, the composition, including a bile salt and at least one peptide selected from the group consisting of cationic peptides represented by SEQ ID NOS: 1 to 34, may be used in mixture with the target protein or the composition and the target protein may be applied in a sequential and separate manner to the epithelium.

In an embodiment of the present disclosure, the composition is adapted to enhance the permeability of target proteins in epithelial tissue.

In the present disclosure, the epithelial tissue is soft tissue covering the inner walls of body organs in direct contact with the outside of the human body, including all mucous membranes. For example, the epithelial tissues include respiratory mucosa covering the nasal cavity and lungs, gastrointestinal mucosa covering the oral cavity, sublingual area, and digestive organs, reproductive mucosa covering the vagina and urethra, excretory mucosa covering the anus, and skin tissue.

No particular limitations are imparted to the target protein of the present disclosure, and various proteins intended for in vivo administration may be selected as delivery targets.

In the present disclosure, the target protein refers to a polymer composed of two or more amino acids linked by peptide bonds.

The delivery effect of composition of the present disclosure on target proteins in epithelial tissue is not limited to cargo proteins of specific sequences.

In one embodiment of the present disclosure, the target protein has a size of 150 kD or less. The composition for protein delivery of the present disclosure is intended to aid the epithelial permeability of the target protein, and the size of the target protein is preferably below a certain size. It was observed that in the case of target proteins having a size of 150 kD or less, the epithelial permeability significantly increases.

In an embodiment of the present disclosure, the target protein may be at least one selected from the group consisting of insulin, insulin analogs, GLP-1 analogs, parathyroid hormone, growth hormone, epidermal growth factor, oligomers, and IgG antibodies, but is not limited thereto. Specifically, for the GLP-1 analogs mentioned above, an example includes liraglutide. In the present disclosure, the bile salt may be a naturally occurring bile salt (e.g., trihydroxy bile salt, dihydroxy bile salt) and may include at least one selected from the group consisting of taurocholate, glycocholate, cholate, taurodeoxycholate, glycodeoxycholate, deoxycholate, chenodeoxycholate, lithocholate, and ursodeoxycholate, but is not limited thereto.

In the present disclosure, when the bile salt is a bile salt conjugated with taurine or glycine (e.g., taurocholate or glycocholate), it can particularly effectively enhance or improve the permeability of a target protein in epithelial tissue.

In the present disclosure, the cationic peptide may be synthesized with a purity of 90% or more for each peptide using conventional peptide synthesis methods known to those skilled in the art, and may be purchased after requesting the production from a peptide manufacturing company.

In the present disclosure, the cationic peptides may include cases where conventional modifications that do not significantly change the properties of the amino acids (e.g., substitution of 1-2 amino acids, substitution between D-form and L-form) have occurred.

In an embodiment of the present disclosure, the peptide as an active ingredient may be selected from the group consisting of variants corresponding to the amino acid sequences represented by SEQ ID NOS: 1 to 34, with a substitution modification occurred on one or two amino acid residues thereof.

In an embodiment of the present disclosure, the composition may further include a fatty acid. As described above, the protein delivery composition of the present disclosure includes a bile salt and a peptide with a specific amino acid sequence as active ingredients, and the inclusion of fatty acids may further enhance the epithelial permeability of the target protein.

In an embodiment of the present disclosure, the fatty acid of the present disclosure may be a C4 to C22 fatty acid. More specifically, the fatty acid may be a C6 to C22 fatty acid, a C8 to C22 fatty acid, a C10 to C22 fatty acid, a C12 to C22 fatty acid, or a C14 to C20 fatty acid.

In a specific embodiment of the present disclosure, the fatty acid of the present disclosure may be a C16 to C18 fatty acid.

The aforementioned fatty acids may be saturated or unsaturated fatty acids, and more preferably, unsaturated fatty acids may be used. The unsaturated fatty acids of the present disclosure mean fatty acids bearing one or more double bonds, and more preferably, at least one of the one or more double bonds included in the unsaturated fatty acids is a cis double bond.

The protein delivery composition according to an embodiment of the present disclosure may further include a phosphate buffer, an acetate buffer, and/or a bicarbonate buffer, but with no limitations thereto.

The composition according to one embodiment of the present disclosure may be applied via transdermal administration, oral administration, sublingual administration, topical application, patch attachment, or inhalation, but with no limitations thereto.

The composition according to an embodiment of the present disclosure may be formulated into patch-type preparations, sprays, ointments, gels, liquids, suppositories, troches, and inhalants through conventional methods known in the art, but with no limitations thereto.

Another aspect of the present disclosure provides a transdermal absorption agent comprising the composition for protein delivery.

The transdermal absorption agent of the present disclosure may further include a target protein that requires delivery, and the target protein is same as described in the foregoing. The transdermal absorption agent of the present disclosure is in a formulation intended to promote the absorption of the target protein through the skin and can be used for various purposes depending on the efficacy of the target protein. Specifically, the transdermal absorption agent of the present disclosure may be used for the purpose of supplementing certain proteins that are deficient in the body to cause specific diseases or symptoms, or for the purpose of delivering proteins into the body to elicit desired effects.

Another aspect of the present disclosure provides a pharmaceutical composition for the prevention or treatment of metabolic diseases, the composition including a bile salt, at least one peptide selected from the group consisting of peptides represented by SEQ ID NOS: 1 to 34, and a protein drug for the prevention or treatment of metabolic diseases as active ingredients.

The bile salt and at least one peptide selected from the group consisting of peptides represented by SEQ ID NOS: 1 to 34, included in the pharmaceutical composition for the prevention or treatment of metabolic diseases of the present disclosure have been described in detail as active ingredients of the protein delivery composition in another aspect of the present disclosure. The pharmaceutical composition of the present disclosure may include a protein component with preventive or therapeutic effects against metabolic diseases as an active ingredient, and the bile salts and peptide with a specific sequence can significantly enhance the epithelial absorption of the protein component within the body.

In an embodiment of the present disclosure, the composition may further include a fatty acid. The fatty acids that can be included in the pharmaceutical composition according to one embodiment of the present disclosure correspond to those that can be included in the protein delivery composition in another aspect of the present disclosure, and the repeated description is omitted.

In one embodiment of the present disclosure, the metabolic diseases may be at least one selected from the group consisting of diabetes, obesity, osteoporosis, and growth hormone deficiency, but are not limited thereto. Specifically, for example, if the pharmaceutical composition of the present disclosure is intended for the prevention or treatment of diabetes, it may include insulin, insulin analogs, or GLP-1 analogs as target proteins. Additionally, if the pharmaceutical composition of the present disclosure is intended for the prevention or treatment of obesity, it may include leptin, pramlintide, GLP-1 receptor antagonists, or liraglutide as target proteins. Furthermore, if the pharmaceutical composition of the present disclosure is intended for the treatment of osteoporosis, it may include parathyroid hormone. By including growth hormone as an active ingredient, the pharmaceutical composition of the present disclosure can help treat growth hormone deficiency or induce growth in subjects in the growth phase. The aforementioned active ingredients are merely specific examples, and one aspect of the present disclosure relates to a pharmaceutical composition characterized by enhanced epithelial delivery efficiency of various protein drugs, with no particular limitations imparted to the types of target proteins as active ingredients in the pharmaceutical composition.

In the present disclosure, the description of the pharmaceutical composition primarily concerns administration to humans, but those skilled in the art will understand that such compositions are generally suitable for administration to all types of animals. Skilled veterinary pharmacologists will understand and be able to design and/or conduct necessary modifications for pharmaceutical compositions for administration to various animals through conventional experimentation if needed.

In the present disclosure, the pharmaceutical composition may be manufactured by any method known in the field of pharmacology or as described hereinbelow. Generally, such manufacturing methods include associating the active ingredient with excipients and/or one or more other auxiliary ingredients, followed by shaping and/or packaging the resulting product into desired single- or multi-dose units if necessary or desired.

In the present disclosure, the pharmaceutical composition may be prepared, packaged, and/or sold in single unit doses and/or multiple single unit doses, and/or may be sold without packaging.

As used herein, the term "unit dose" refers to an individual quantity of a pharmaceutical composition that contains a predetermined amount of active ingredient. The amount of active ingredient is generally equivalent to the dosage of the active ingredient administered to a subject and/or a convenient fraction of such dosage, such as half or one-third of the dosage.

In the present disclosure, the relative amount of the active ingredient, pharmaceutically acceptable excipients, and/or any additional ingredients in the pharmaceutical composition will vary depending on the identity, size, and/or condition of the subject being treated and the route of administration of the composition. Specifically, for example, a pharmaceutical composition according to one embodiment of the present disclosure may contain 0.1% to 100% (w/w) of the active ingredient.

In the present disclosure, pharmaceutically acceptable excipients include any solvent, dispersion medium, diluent, or other liquid vehicle, dispersion or suspension aid, surfactant, isotonic agent, thickener or emulsifier, preservative, solid binder, lubricant, etc., suitable for the specific dosage form purpose. Remington's literature [The Science and Practice of Pharmacy, 21st Edition, A. R. Gennaro, (Lippincott, Williams & Wilkins, Baltimore, MD, 2006)] discloses various excipients and known techniques for preparing pharmaceutical compositions. The use of any conventional carrier medium is considered to be within the scope of the present disclosure, except that it is incompatible with a substance or its derivative, for example, by providing any unwanted biological effect or otherwise interacting with any other component of pharmaceutical compositions in a harmful manner. Pharmaceutically acceptable excipients are at least 95%, 96%, 97%, 98%, 99%, or 100% pure.

The excipients are approved for human and veterinary use. In some embodiments, the excipients are approved by the U.S. Food and Drug Administration (FDA). In some embodiments, the excipients are of pharmaceutical grade. In some embodiments, the excipients meet the standards of the United States Pharmacopeia (USP), European Pharmacopeia (EP), British Pharmacopeia, and/or International Pharmacopeia (EP). In some embodiments, the excipients are approved for human and veterinary use.

Pharmaceutically acceptable excipients used in the preparation of pharmaceutical compositions include, but are not limited to, inert diluents, dispersants and/or granulating agents, surfactants and/or emulsifiers, disintegrants, binders, preservatives, buffers, lubricants, and/or oils.

These excipients may optionally be included in the formulations of the present disclosure. Excipients such as cocoa butter and suppository wax, colorants, coatings, sweeteners, flavorings, and perfumes may be present in the compositions at the discretion of the formulator.

Exemplary diluents include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dried starch, corn starch, powdered sugar, and combinations thereof.

Another aspect of the present disclosure provides a health functional food composition including a bile salt, at least one peptide selected from the group consisting of peptides represented by SEQ ID NOS: 1 to 34, and a health-functional protein as active ingredients.

In an embodiment of the present disclosure, the composition of the present disclosure may further include a fatty acid. The fatty acids included in the health functional food composition according to an embodiment of the present disclosure correspond to those included in the protein delivery composition according to another aspect of the present disclosure.

The health functional food composition of the present disclosure can include sitologically acceptable protein components as various active ingredients and can have preventive or ameliorative effects on various diseases or physical function abnormalities.

The health functional food composition of the present disclosure includes ingredients typically added during food manufacturing, such as proteins, carbohydrates, fats, nutrients, and flavoring agents. For instance, when manufactured as a drink, the composition may include flavoring agents or natural carbohydrates in addition to the active ingredients. Examples of natural carbohydrates include monosaccharides (e.g., glucose, fructose, etc.); disaccharides (e.g., maltose, sucrose, etc.); oligosaccharides; polysaccharides (e.g., dextrin, cyclodextrin, etc.); and sugar alcohols (e.g., xylitol, sorbitol, erythritol, etc.).

As flavoring agents, natural flavorings (e.g., thaumatin, stevia extract, etc.) and synthetic flavorings (e.g., saccharin, aspartame, etc.) may be used.

Another aspect of the present disclosure provides a cosmetic composition including a bile salt, at least one peptide selected from the group consisting of peptides represented by SEQ ID NOS: 1 to 34, and a functional protein as active ingredients.

In an embodiment of the present disclosure, the composition may further include a fatty acid. The fatty acids included in the cosmetic composition according to one embodiment of the present disclosure correspond to those included in the protein delivery composition according to another aspect of the present disclosure.

The cosmetic composition of the present disclosure may include cosmetologically acceptable protein components as various active ingredients and can have preventive or ameliorative effects on various diseases or physical function abnormalities. These effects are not limited to diseases or physical function abnormalities observed on the epithelium. As long as the present disclosure provides transdermal permeability, the composition can be applied to a broader range of diseases or physical function abnormalities.

The cosmetic composition of the present disclosure may be formulated into any form commonly manufactured in the industry, such as solutions, suspensions, emulsions, pastes, gels, creams, lotions, powders, soaps, surfactant-containing cleansers, oils, powder foundations, emulsion foundations, wax foundations, and sprays, but with no limitations thereto. More specifically, the composition may be formulated as softening lotions, nourishing lotions, lotions, nourishing creams, massage creams, essences, eye creams, cleansing creams, cleansing foams, cleansing waters, packs, sprays, or powders.

When the formulation of the present disclosure is a paste, cream, lotion, or gel, use may be made of animal oils, vegetable oils, waxes, paraffin, starch, tragacanth, cellulose derivatives, polyethylene glycol, silicones, bentonite, silica, talc, or zinc oxide as a carrier ingredient.

When the formulation of the present disclosure is a powder or spray, the carrier ingredient may be lactose, talc, silica, aluminum hydroxide, calcium silicate, or polyamide powder. For a spray, the formulation may additionally include a propellant such as chlorofluorohydrocarbons, propane/butane, or dimethyl ether.

When the formulation of the present disclosure is a solution or emulsion, the carrier ingredients may include solvents, solubilizers, or emulsifiers, such as water, ethanol, isopropanol, ethyl carbonate, ethyl acetate, benzyl alcohol, benzyl benzoate, propylene glycol, 1,3-butylene glycol oil, glycerol aliphatic esters, polyethylene glycol, or sorbitan fatty acid esters.

When the formulation of the present disclosure is a suspension, the carrier ingredients may include liquid diluents such as water, ethanol, or propylene glycol, suspending agents such as ethoxylated isostearyl alcohol, polyoxyethylene sorbitol esters, and polyoxyethylene sorbitan esters, microcrystalline cellulose, aluminum metahydroxide, bentonite, agar, or tragacanth.

When the formulation of the present disclosure is a surfactant-containing cleanser, the carrier ingredients may include aliphatic alcohol sulfates, aliphatic alcohol ether sulfates, sulfosuccinate monoesters, isethionates, imidazolinium derivatives, methyltaurates, sarcosinates, fatty acid amide ether sulfates, alkylamido betaines, aliphatic alcohols, fatty acid glycerides, fatty acid diethanolamides, vegetable oils, lanolin derivatives, or ethoxylated glycerol fatty acid esters.

The ingredients included in the cosmetic composition of the present disclosure, besides the active ingredients and carrier ingredients, include components commonly used in cosmetic compositions, such as antioxidants, stabilizers, solubilizers, vitamins, pigments, and fragrances.

### Advantageous Effects of Invention

(a) The present disclosure provides a composition for protein delivery with enhanced permeability in epithelial tissue.
(b) The present disclosure provides a transdermal absorption agent comprising the composition for protein delivery.
(c) The present disclosure provides a pharmaceutical composition for the prevention or treatment of metabolic diseases, comprising the composition for protein delivery.
(d) The present disclosure provides a health functional food composition comprising the composition for protein delivery.
(e) The present disclosure provides a cosmetic composition comprising the composition for protein delivery.
(f) When using the protein delivery composition of the present disclosure, the permeability of macromolecular target proteins through epithelial and transdermal tissues is increased, enabling the effective delivery of target proteins into the body in non-invasive manners (non-injection formulations).

Therefore, the composition can be used for the prevention or treatment of metabolic diseases, including diabetes and obesity, and has the advantage of being applicable to cosmetics and health functional foods.

### Brief Description of Drawings

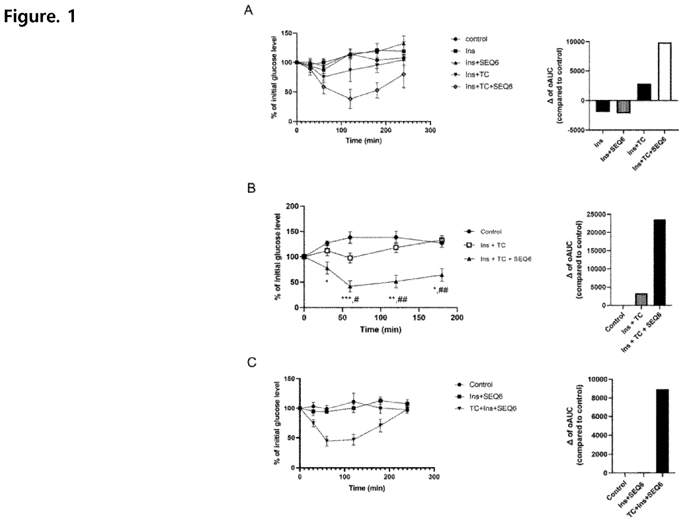

FIG. 1 illustrates to the change in insulin absorption in epithelial tissue due to bile salts and/or cationic peptides. The degree of insulin absorption in the body is indicated by the blood glucose-lowering effect after administration. When both bile salt (e.g., taurocholate) and cationic peptide (e.g., SEQ6) are included, insulin absorption in skin mucosal tissue significantly increases compared to when neither bile salts nor cationic peptides are included or when only one of bile salts (TC: taurocholate, FIG. 1A) or cationic peptides (SEQ6, FIG. 1A) is included. Similarly, insulin absorption in sublingual mucosal tissue significantly increases compared to when neither bile salt nor cationic peptides are included or when only one of bile salts (TC: taurocholate, FIG. 1B) or cationic peptides (SEQ6, FIG. 1C) is included.
FIG. 2 illustrates the change in insulin absorption upon oral administration of bile salt and/or cationic peptides. The degree of insulin absorption in the body is indicated by the blood glucose-lowering effect after administration. When cationic peptides (SEQ6, FIG. 2A, SEQ2, FIG. 2B) are mixed with bile salts, insulin absorption significantly increases in a dose-dependent manner of the cationic peptides.
FIG. 3 shows the insulin absorption rate in sublingual mucosal tissue of different types of cationic peptides mixed with bile salt. The degree of insulin absorption in the body is indicated by the blood glucose-lowering effect after administration.
FIG. 4 shows the insulin absorption rate in sublingual mucosal tissue depending on bile salts and cationic peptides composed of D-form or L-form amino acids. The degree of insulin absorption in the body is indicated by the blood glucose-lowering effect after administration.
FIG. 5 shows the insulin absorption rate in sublingual mucosal tissue depending on the mixture of different types of bile salts and cationic peptides. The degree of insulin absorption in the body is indicated by the blood glucose-lowering effect after administration (TC: taurocholate, GC: glycocholate, CdOC: chenodeoxycholate, dOC: deoxycholate).
FIG. 6 shows the absorption rate of various insulin analogs in sublingual mucosal tissue. The degree of insulin absorption in the body is indicated by the blood glucose-lowering effect after administration.
FIG. 7 shows the change in insulin absorption rate in sublingual mucosal tissue (A) and the blood glucose-lowering effect due to insulin absorption upon oral administration (B) in a type 1 diabetes mouse model. A composition containing 30 mM taurocholate and 1 µM SEQ6 was used as the absorption enhancer.
FIG. 8 shows the change in insulin absorption rate upon skin application of a mixture of bile salt (taurocholate) and cationic peptides (SEQ6). The degree of insulin absorption in the body is indicated by the change in blood glucose after administration.
FIG. 9 shows the absorption rate of liraglutide in sublingual mucosal tissue due to a mixture of bile salt (taurocholate) and cationic peptides (SEQ6).

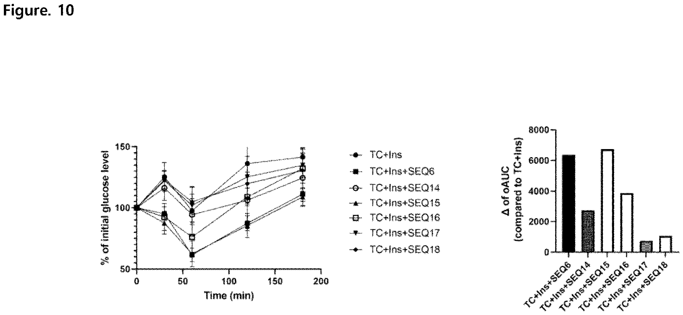

FIG. 10 shows the insulin absorption rate in sublingual mucosal tissue depending on cationic peptides represented by different amino acid sequences. The degree of insulin absorption in the body is indicated by the blood glucose-lowering effect after administration.
FIG. 11 shows the absorption rate of epidermal growth factor in nasal mucosal tissue due to a mixture of bile salt (taurocholate) and cationic peptides (SEQ14).
FIG. 12 shows the absorption rate of human growth hormone in nasal mucosal tissue due to a mixture of bile salt (taurocholate) and cationic peptides.
FIG. 13 shows the change in the absorption of human antibody (IgG) in nasal mucosal tissue due to a mixture of bile salt (taurocholate) and cationic peptide, measured by the plasma concentration of hIgG 3 hours after administration in mice (A), and compares the plasma concentration of nasal administration with subcutaneous injection of the same dose (B).

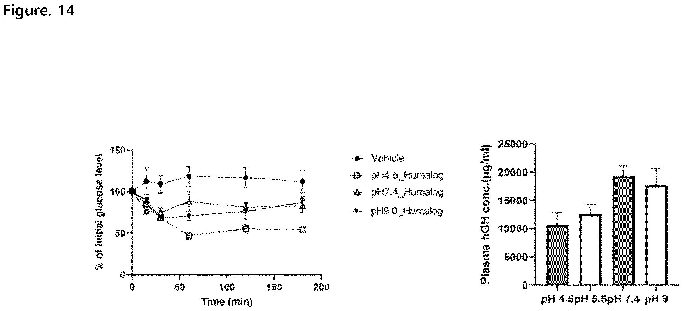

FIG. 14 shows the effect of pH on the protein delivery efficiency of a mixture of bile salt (taurocholate) and cationic peptide.
FIG. 15 shows the change in insulin absorption rate in nasal mucosal tissue due to a mixture of bile salt (taurocholate), fatty acid (oleic acid), and cationic peptide. The change in blood glucose (A) and the change in insulin (B) after administration are indicated.

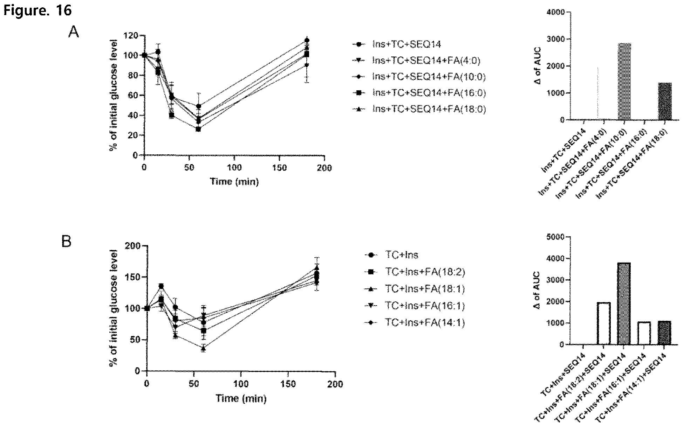

FIG. 16 shows the change in insulin delivery efficiency due to the mixture of bile salt (taurocholate) and cationic peptide with various fatty acids. It was confirmed that saturated fatty acids (FIG. 16A) and unsaturated fatty acids (FIG. 16B) can cause changes in delivery efficiency depending on the chain length and the presence of double bonds.

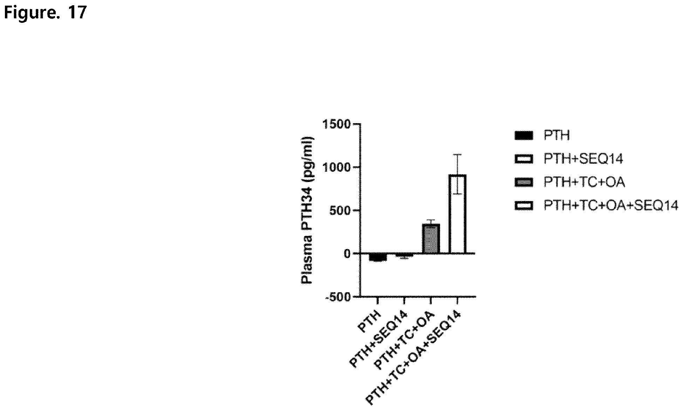

FIG. 17 shows the plasma concentration of parathyroid hormone 34 (PTH) 15 minutes after administration in nasal mucosal tissue due to mixtures of bile salt (taurocholate), fatty acid (oleic acid), and cationic peptide (SEQ14).

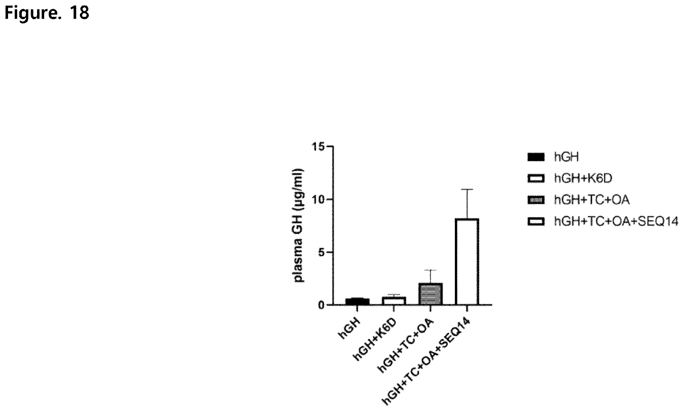

FIG. 18 shows the plasma concentration of human growth hormone (hGH) 15 minutes after administration in nasal mucosal tissue due to mixtures of bile salt (taurocholate), fatty acid (oleic acid), and cationic peptide (SEQ14).

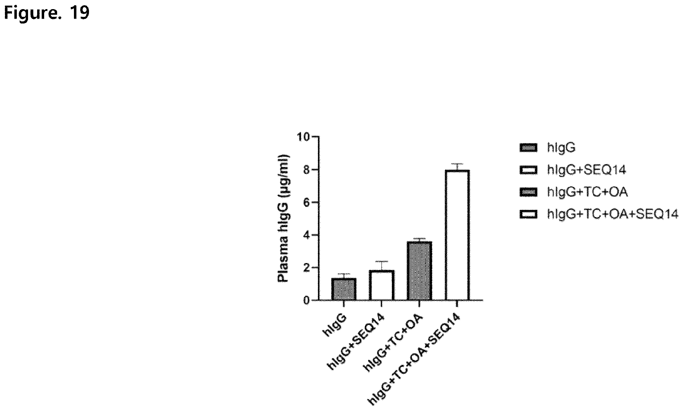

FIG. 19 shows the plasma concentration of human antibody IgG (hIgG) 3 hours after administration in nasal mucosal tissue due to mixtures of bile salt (taurocholate, TC), fatty acid (oleic acid, OA), and cationic peptide (SEQ14).

### Best Mode for Carrying out the Invention

Below, a detailed description will be given of the present disclosure.

Unless otherwise defined, all technical and scientific terms used herein have the same meaning as commonly understood by one of ordinary skill in the art to which this invention belongs. Generally, the nomenclature used herein is well-known and commonly used in the art.

### EXAMPLES

### EXAMPLE 1: Confirmation of Increased Insulin Absorption in Sublingual Mucosal Tissue by Bile Salts and Cationic Peptides

Examination was made of the change in insulin absorption in sublingual mucosal tissue due to bile salts and/or peptides represented by SEQ ID NOS: 1 to 18. All cationic peptides used in the experiment were synthesized by AnyGen Co., Ltd. The absorption rate of insulin was indicated by measuring the blood glucose-lowering rate after insulin administration. The experimental groups were as follows:
1) TC: 100 mM Taurocholate (Sigma-Aldrich Korea, Korea), 2) Ins+TC: 100 mM Taurocholate + 2U/kg dose of insulin (Humulin N, Korea Lilly, Korea), 3) Ins+TC+SEQ6: 100 mM Taurocholate + 2U/kg dose of insulin + 1 µM SEQ6, 4) SEQ6: 1 µM SEQ6, 5) Ins+SEQ6: 1 µM SEQ6 + 2U/kg dose of insulin, and 6) TC+Ins+SEQ6: 100 mM Taurocholate + 1 µM SEQ6 + 2U/kg dose of insulin

Specifically, C57BL/6 mice (Orient Bio, Korea) maintaining normal blood glucose levels were anesthetized by intraperitoneal injection of Ketamine/Xylazine (100 mg/kg and 10 mg/kg, respectively) (BK Pharm, Korea). After confirming stable anesthesia, blood glucose levels were measured using an Accu-Chek glucometer before the administration of bile salts and/or cationic peptides along with insulin. Each experimental group received 10 µl of the solution administered to the sublingual mucosa, and blood glucose changes were measured over time.

As a result, the blood glucose-lowering rate by insulin was low when taurocholate or SEQ6 was administered alone, but significantly increased when both taurocholate and SEQ6 were administered together (FIGS. 1A and 1B). This indicates that the combination of taurocholate and SEQ6 significantly increases insulin absorption through the sublingual mucosal tissue.

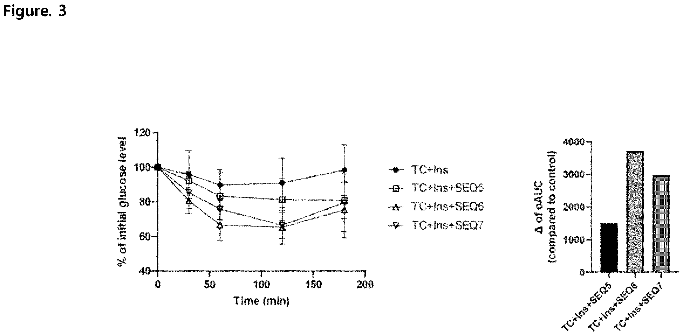

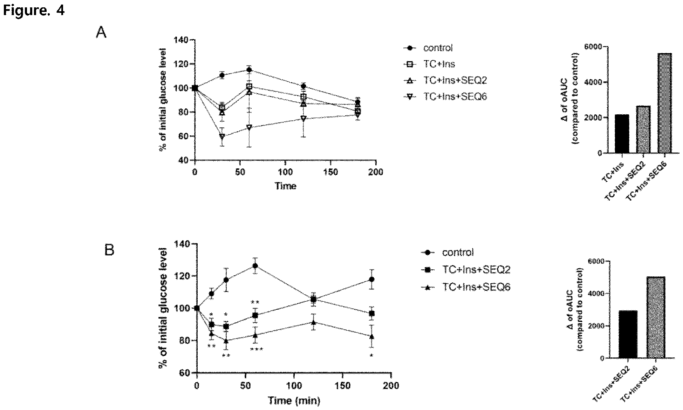

It was observed that all cationic peptides increased insulin absorption to varying degrees depending on the chain length of the cationic peptide (FIG. 3). Additionally, insulin absorption was found to be more effective with D-form amino acids than with L-form amino acids in the cationic peptides (FIG. 4).

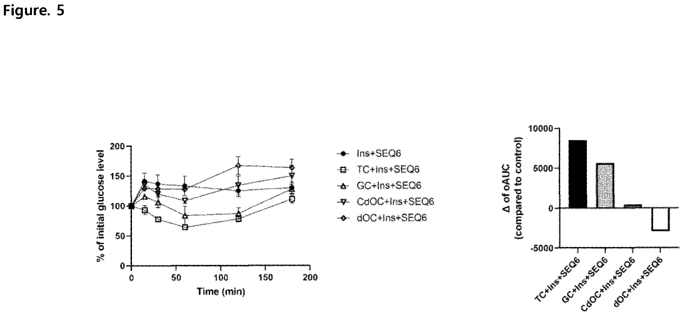

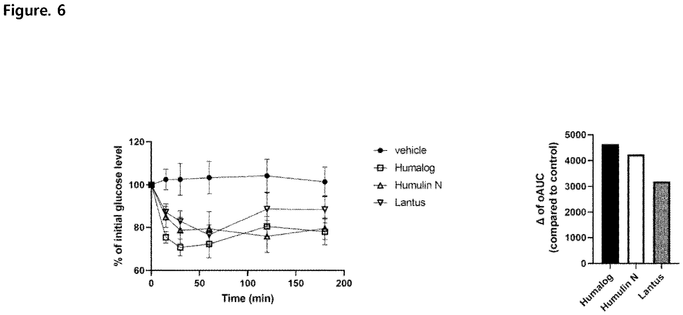

Depending on the type of bile salt, the degree of insulin absorption varied, but all increased insulin absorption (FIG. 5). It was also confirmed that bile salts and cationic peptides increased the absorption of various insulin analogs in addition to insulin (FIG. 6).

### EXAMPLE 2: Confirmation of Increased Insulin Absorption Upon Oral Administration of Bile Salts and Cationic Peptides

Examination was made of the change in insulin absorption upon oral administration of bile salts and/or peptides represented by SEQ ID NOS: 1 to 18. The absorption rate of insulin was indicated by measuring the blood glucose-lowering rate after insulin administration. The experimental groups were as follows:
1) Ins+TC: 100 mM Taurocholate + 2U/kg dose of insulin, 2) Ins+TC+SEQ6: 100 mM Taurocholate + 2U/kg dose of insulin + 10 µM SEQ6, 3) Ins+TC+SEQ6: 100 mM Taurocholate + 2U/kg dose of insulin + 1 µM SEQ6, 4) Ins+TC+SEQ6: 100 mM Taurocholate + 2U/kg dose of insulin + 0.1 µM SEQ6, 5) Ins+TC+SEQ2: 100 mM Taurocholate + 2U/kg dose of insulin + 100 µM SEQ2, 6) Ins+TC+SEQ2: 100 mM Taurocholate + 2U/kg dose of insulin + 10 µM SEQ2, and 7) Ins+TC+SEQ6: 100 mM Taurocholate + 2U/kg dose of insulin + 1 µM SEQ2

Specifically, C57BL/6 mice maintaining normal blood glucose levels were prepared, and blood glucose levels were measured using an Accu-Chek glucometer before administering insulin with bile salts and/or cationic peptides. Insulin, along with bile salts and/or cationic peptides, was administered directly into the stomach using a zonde, and blood glucose changes were measured over time.

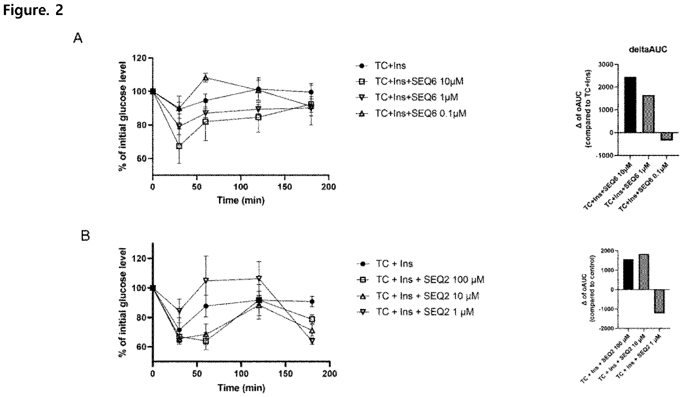

As a result, the blood glucose-lowering effect of insulin was observed to be dependent on the dose of cationic peptides (FIGS. 2A and 2B).

### EXAMPLE 3: Confirmation of Increased Insulin Absorption in Type 1 Diabetic Mouse Model Due to Bile Salts and Cationic Peptides

Examination was made of the change in insulin absorption in sublingual mucosal tissue due to bile salts and/or peptides represented by SEQ ID NOS 1 to 18 in a Type 1 diabetic mouse model treated with Streptozotocin. Specifically, C57BL/6 mice to be used in the experiment were administered streptozotocin (Sigma-Aldrich Korea, Korea) dissolved in citrate buffer (100 mM, pH 4.5) at a dose of 150 mg/kg, and after 6 hours, they were allowed free access to food. Blood glucose levels were measured every morning at the same time, and mice showing blood glucose levels of 400 mg/dl or higher for more than 3 days were considered diabetic and used in the experiment.

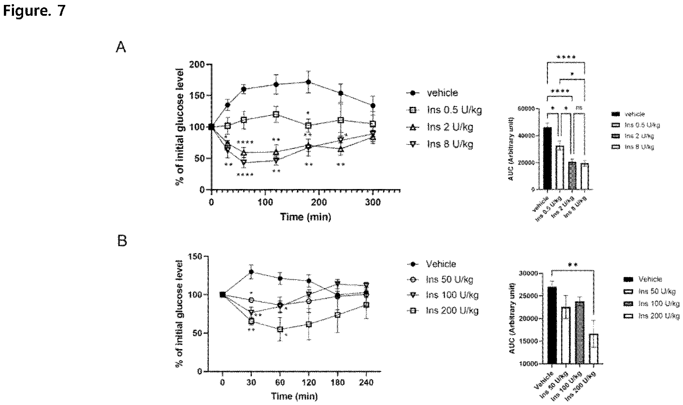

The results showed that when bile salts and cationic peptides were administered with insulin in the sublingual mucosal tissue (FIG. 7A) and via oral administration (FIG. 7B), blood glucose levels were effectively reduced within similar concentration ranges to those of normal glucose mice (FIG. 7).

### EXAMPLE 4: Confirmation of Increased Insulin Absorption in Type 1 Diabetic Mouse Model Due to Bile Salts and Cationic Peptides

Examination was made of the change in insulin absorption upon topical application due to bile salts and/or peptides represented by SEQ ID NO: 1 to SEQ ID NO: 18. The absorption rate of insulin was indicated by measuring the blood glucose-lowering rate after insulin administration. Specifically, C57BL/6 mice maintaining normal blood glucose levels were shaved on the back using a depilatory cream the day before the experiment. On the day of the experiment, the mice were anesthetized by intraperitoneal injection of Ketamine/Xylazine (100 mg/kg and 10 mg/kg, respectively). After confirming stable anesthesia, blood glucose levels were measured using an Accu-Chek glucometer before administering insulin with bile salts and/or cationic peptides. A 50 µl volume of the solution was applied to the shaved skin, and blood glucose changes were measured over time.

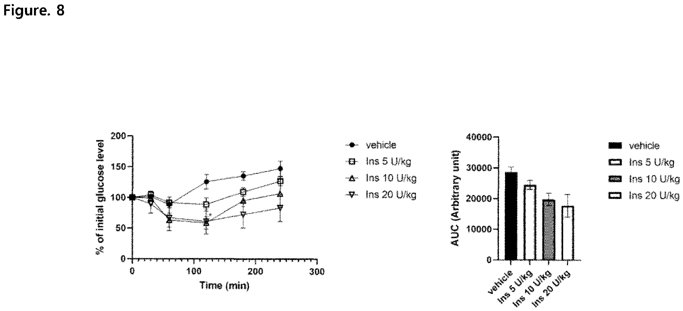

The results showed that the excellent blood glucose-lowering effect of insulin was observed in a dose-dependent manner upon topical application of insulin (FIG. 8) .

### EXAMPLE 5: Confirmation of Increased Liraglutide Absorption in Sublingual Mucosal Tissue Due to Bile Salts and Cationic Peptides

Examination was made of the change in liraglutide (Victoza Pen, Novo Nordisk Pharmaceuticals, Korea) absorption in sublingual mucosal tissue due to bile salts and/or peptides represented by SEQ ID NOS: 1 to 18. The absorption rate of liraglutide was indicated by measuring the improvement in glucose tolerance due to liraglutide. Specifically, C57BL/6 mice maintaining normal blood glucose levels were anesthetized by intraperitoneal injection of Ketamine/Xylazine (100 mg/kg and 10 mg/kg, respectively). After confirming stable anesthesia, blood glucose levels were measured using an Accu-Chek glucometer before administering liraglutide with bile salts and/or cationic peptides. A 10 µl volume of liraglutide was administered to the sublingual mucosa under the tongue, followed by an intraperitoneal injection of 1 g/kg glucose. Blood glucose changes were then measured over time.

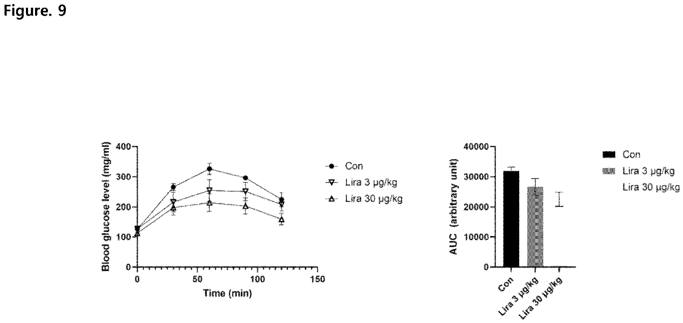

The results showed that excellent glucose tolerance improvement effects of liraglutide were observed in a dose-dependent manner (FIG. 9).

### EXAMPLE 6: Confirmation of Increased Absorption of Epidermal Growth Factor in Nasal Mucosal Tissue Due to Bile Salts and Cationic Peptides

Examination was made of the change in absorption of epidermal growth factor (EGF) in nasal mucosal tissue due to bile salts and/or peptides represented by SEQ ID NOS: 1 to 34. All cationic peptides used in the experiment were synthesized by AnyGen Co., Ltd. The epidermal growth factor used was recombinant human EGF from GIBCO (USA). The absorption rate of EGF was indicated by measuring the concentration of EGF in plasma after administration. Plasma was separated from blood collected from the retro-orbital plexus and measured using a human ELISA kit (R&D Systems, USA). The experimental groups were as follows:

1) EGF: EGF dissolved in 100 mM acetate buffer, pH 4.5, administered nasally at 30 µg/kg, 2) EGF+SEQ14: The same amount of EGF administered nasally with 10 µM SEQ14 in the same buffer, and 3) EGF+TC+SEQ14: The same amount of EGF administered nasally with 30 mM taurocholate and 10 µM SEQ14 in the same buffer.

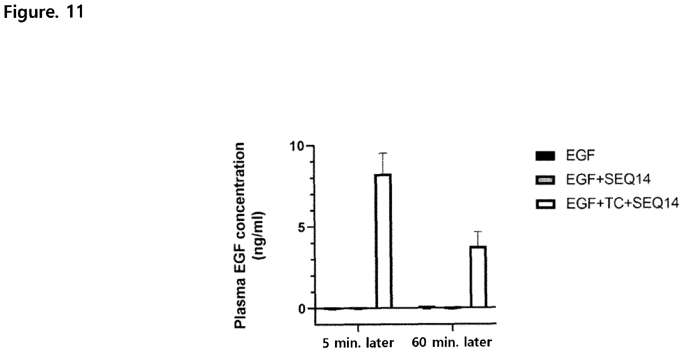

Specifically, 8-week-old C57BL/6 mice (Orient Bio, Korea) were anesthetized by intraperitoneal injection of Ketamine/Xylazine (100 mg/kg and 10 mg/kg, respectively) (BK Pharm, Korea). After confirming stable anesthesia, each experimental group received 10 µl of the solution administered directly into the nostrils. Blood was collected from the retro-orbital plexus using heparin-coated capillaries at 5 and 60 minutes post-administration, and plasma was separated. Measurements were taken following the manufacturer's manual for the ELISA kit and represented graphically (FIG. 11). The results showed that, similar to insulin, plasma levels of EGF significantly increased when taurocholate and SEQ14 were administered together.

### EXAMPLE 7: Confirmation of Increased Absorption of Human Growth Hormone in Nasal Mucosal Tissue Due to Bile Salts and Cationic Peptides

Examination was made of the change in absorption of human growth hormone (hGH, 22 kDa) in nasal mucosal tissue due to bile salts and/or peptides represented by SEQ ID NOS: 1 to 34. All cationic peptides used in the experiment were synthesized by AnyGen Co., Ltd. The human growth hormone used was Eutropin Injection from LG Chem. The absorption rate of the administered protein was indicated by measuring the concentration of the administered protein in plasma after administration. Plasma was separated from blood collected from the retro-orbital plexus and measured using a human Growth Hormone ELISA kit (R&D Systems, USA). The experimental groups were as follows:
1) hGH: Human growth hormone dissolved in 100 mM acetate buffer, pH 4.5, administered nasally at 330 µg/kg, 2) hGH+SEQ14: The same amount of hGH administered nasally with 10 µM SEQ14 in the same buffer, 3) hGH+TC: The same amount of hGH administered nasally with 30 mM taurocholate in the same buffer, 4) hGH+TC+SEQ14: The same amount of hGH administered nasally with 30 mM taurocholate and 10 µM SEQ14 in the same buffer

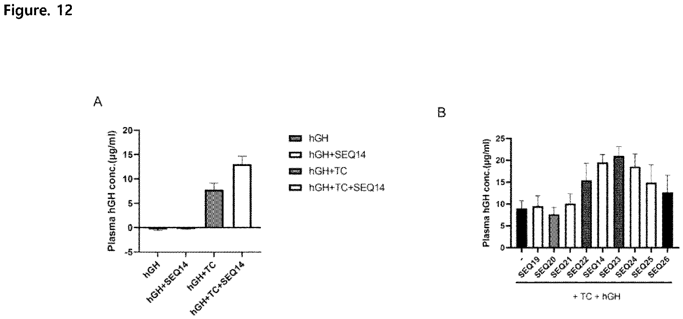

Specifically, 8-week-old C57BL/6 mice (Orient Bio, Korea) were anesthetized by intraperitoneal injection of Ketamine/Xylazine (100 mg/kg and 10 mg/kg, respectively) (BK Pharm, Korea). After confirming stable anesthesia, each experimental group received 10 µl of the solution administered directly into the nostrils. Blood was collected from the retro-orbital plexus using heparin-coated capillaries at 15 minutes post-administration, and plasma was separated. Measurements were taken following the manufacturer's manual for the ELISA kit and represented graphically (FIG. 12A). The results showed that plasma levels of GH significantly increased when taurocholate and SEQ14 were administered nasally together. Additionally, to further confirm the delivery efficiency improvement of cationic peptides SEQ19 to SEQ26, experimental groups were prepared by substituting SEQ19 to SEQ26 for the peptide in group 4. The results showed significant improvement effects with peptides SEQ22 to SEQ26 (FIG. 12B).

### EXAMPLE 8: Confirmation of Increased Absorption of Human Antibody IgG in Nasal Mucosal Tissue Due to Bile Salts and Cationic Peptides

Examination was made of the change in absorption of human antibody IgG (hIgG, 150 kDa) in nasal mucosal tissue due to bile salts and/or peptides represented by SEQ ID NOS: 1 to 34. All cationic peptides used in the experiment were synthesized by AnyGen Co., Ltd. Human antibody IgG was purchased from Sigma-Aldrich Korea. The absorption rate of the administered protein was indicated by measuring the concentration of the administered protein in plasma after administration. Plasma was separated from blood collected from the retro-orbital plexus and measured for protein levels using a human IgG total ELISA kit from Invitrogen (USA). The experimental groups were as follows:
1) hIgG: Human IgG dissolved in 100 mM acetate buffer, pH 4.5, administered nasally at 3.3 mg/kg, 2) hIgG+SEQ14: The same amount of hIgG administered nasally with 10 µM SEQ14 in the same buffer, 3) hIgG+TC: The same amount of hIgG administered nasally with 30 mM taurocholate in the same buffer, and 4) hIgG+TC+SEQ14: The same amount of hIgG administered nasally with 30 mM taurocholate and 10 µM SEQ14 in the same buffer

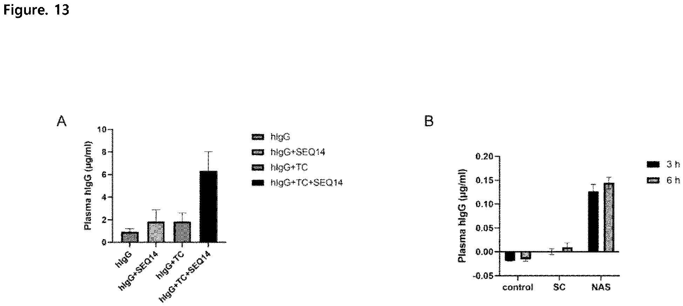

Specifically, 8-week-old C57BL/6 mice (Orient Bio, Korea) were anesthetized by intraperitoneal injection of Ketamine/Xylazine (100 mg/kg and 10 mg/kg, respectively) (BK Pharm, Korea). After confirming stable anesthesia, each experimental group received 10 µl of the solution administered directly into the nostrils. Blood was collected from the retro-orbital plexus using heparin-coated capillaries at 3 hours post-administration, and plasma was separated. Measurements were taken following the manufacturer's manual for the ELISA kit and represented graphically (FIG. 13). The results showed that plasma levels of hIgG significantly increased when taurocholate and SEQ14 were administered together. Additionally, to compare the efficiency of nasal administration of hIgG with injection, the experimental groups were divided into non-treated control, subcutaneous injection of hIgG (SC), and nasal administration of hIgG. Blood was collected at 3 and 6 hours post-administration. For subcutaneous injection, hIgG was administered with PBS. The results showed a significantly higher increase in blood IgG levels with nasal administration compared to subcutaneous injection (FIG. 13B).

### EXAMPLE 9: Confirmation of Increased Insulin Absorption in Nasal Mucosal Tissue Due to Bile Salts, Fatty Acids, and Cationic Peptides

It is known that bile salts form micelles at certain concentrations, and larger micelles are formed when mixed with fatty acids. This experiment was conducted to examine whether the significant protein absorption enhancement effect of bile salts and cationic peptides also appears in compositions containing bile salts, fatty acids, and cationic peptides. The experiment involved measuring blood glucose levels after nasal administration of insulin. Oleic acid, purchased from Sigma-Aldrich Korea, was used as the fatty acid. The experimental groups were as follows:
1) Control: 15 mM taurocholate, 10 µM SEQ14 dissolved in 100 mM acetate buffer, pH 4.5, administered nasally, 2) Ins: Insulin at a dose of 1 U/kg dissolved in 100 mM acetate buffer, pH 4.5, administered nasally, 3) Ins+SEQ14: Insulin at a dose of 1 U/kg, 10 µM SEQ14 dissolved in 100 mM acetate buffer, pH 4.5, administered nasally, 4) Ins+TC+OA: Insulin at a dose of 1 U/kg, 15 mM taurocholate, 5 mM oleic acid dissolved in 100 mM acetate buffer, pH 4.5, administered nasally, and 5) Ins+TC+OA+SEQ14: Insulin at a dose of 1 U/kg, 15 mM taurocholate, 5 mM oleic acid, 10 µM SEQ14 dissolved in 100 mM acetate buffer, pH 4.5, administered nasally

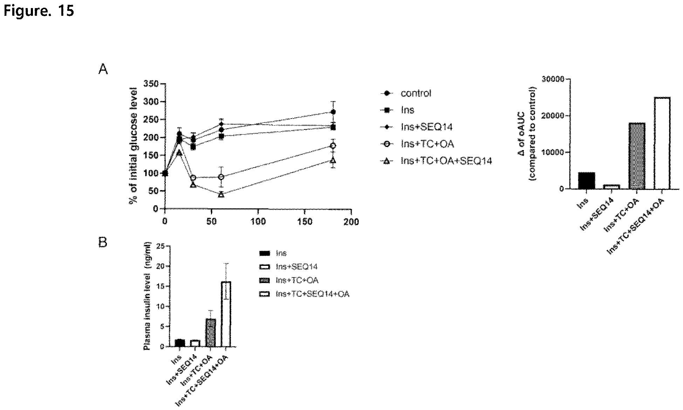

Specifically, C57BL/6 mice maintaining normal blood glucose levels were prepared, and blood glucose was measured using an Accu-Chek glucometer before administering the experimental solutions. Each experimental group solution was administered directly into one nostril of the mouse using a micropipette at a volume of 10 µl, and blood glucose changes were measured over time. The results showed a significant blood glucose-lowering effect in the mixed composition of bile salts, fatty acids, and cationic peptides (FIG. 15A). Under the same experimental conditions, plasma was collected 15 minutes post-administration and measured using a human insulin ELISA (ALPCO, USA), showing a more pronounced increase (FIG. 15B).

### EXAMPLE 10: Confirmation of Increased Insulin Absorption in Nasal Mucosal Tissue Due to Different Types of Fatty Acids in Bile Salts, Fatty Acids, and Cationic Peptides Compositions

To examine effects of different types of fatty acids, the experimental groups were composed of: 1) Insulin at a dose of 1 U/kg, 15 mM taurocholate, and 5 mM oleic acid dissolved in 100 mM acetate buffer, pH 4.5, administered nasally, and optionally plus 5 mM other fatty acids, administered nasally. The fatty acids used in the experiment were purchased from Sigma-Aldrich. Saturated fatty acids included butyric acid (4:0), decanoic acid (10:0), palmitic acid (16:0), and stearic acid (18:0). Unsaturated fatty acids included myristoleic acid (14:1), palmitoleic acid (16:1), oleic acid (18:1), and linoleic acid (18:2). Specifically, C57BL/6 mice maintaining normal blood glucose levels were prepared, and blood glucose was measured using an Accu-Chek glucometer before administering the experimental solutions. Each experimental group solution was administered directly into one nostril of the mouse using a micropipette at a volume of 10 µl, and blood glucose changes were measured over time. The results showed that C16 to C18 chain length fatty acids, especially unsaturated fatty acids, provided the best insulin delivery when included in compositions of bile salts and cationic peptides compared to saturated fatty acids.

## Claims

1. A composition for protein delivery, comprising a bile salt and at least one peptide selected from the group consisting of peptides represented by SEQ ID NOS: 1 to 34 as active ingredients.

2. The composition of claim 1, wherein the composition enhances permeability of a target protein in epithelial tissue.

3. The composition of claim 2, wherein the target protein has a size of 150 kD or less.

4. The composition of claim 2, wherein the target protein comprises at least one selected from the group consisting of insulin, insulin analogs, GLP-1 analogs, parathyroid hormone, growth hormone, epidermal growth factor, oligomers, and IgG antibodies.

5. The composition of claim 1, wherein the bile salt comprises at least one selected from the group consisting of taurocholate, glycocholate, cholate, taurodeoxycholate, glycodeoxycholate, deoxycholate, chenodeoxycholate, lithocholate, and urosodeoxycholate.

6. The composition of claim 1, wherein the peptide is selected from the group consisting of variants corresponding to the amino acid sequences represented by SEQ ID NOS: 1 to 34, with a substitution modification occurred on one or two amino acid residues thereof.

7. The composition of claim 1, further comprising a fatty acid.

8. The composition of claim 7, wherein the fatty acid is a C4 to C22 fatty acid.

9. The composition of claim 8, wherein the fatty acid is a C16 to C18 fatty acid.

10. A transdermal absorption agent comprising the composition for protein delivery according to any one of claims 1 to 9.

11. A pharmaceutical composition for preventing or treating metabolic diseases, the composition comprising a bile salt, at least one peptide selected from the group consisting of peptides represented by SEQ ID NOS: 1 to 34, and a protein drug for preventing or treating metabolic diseases as active ingredients.

12. The pharmaceutical composition of claim 11, further comprising a fatty acid.

13. The pharmaceutical composition of claim 11, wherein the metabolic disease is selected from the group consisting of diabetes, obesity, osteoporosis, and growth hormone deficiency.

14. A health functional food composition comprising a bile salt, at least one peptide selected from the group consisting of peptides represented by SEQ ID NOS: 1 to 34, and a functional protein as active ingredients.

15. The health functional food composition of claim 14, further comprising a fatty acid.

16. A cosmetic composition comprising a bile salt, at least one peptide selected from the group consisting of peptides represented by SEQ ID NOS: 1 to 34, and a functional protein as active ingredients.

17. The cosmetic composition of claim 16, further comprising a fatty acid.
